(19) Europäisches Patentamt
European Patent Office
Office européen des brevets

(11) Veröffentlichungsnummer: 0 336 211
B1

(12) # EUROPÄISCHE PATENTSCHRIFT

(45) Veröffentlichungstag der Patentschrift: **07.11.90**

(51) Int. Cl.[5]: **C07C 69/736**, C07C 69/738, A01N 37/38

(21) Anmeldenummer: **89105090.8**

(22) Anmeldetag: **22.03.89**

(54) **Ortho-substituierte Phenolether und Fungizide, die diese Verbindungen enthalten.**

(30) Priorität: **31.03.88 DE 3811012**

(43) Veröffentlichungstag der Anmeldung:
**11.10.89 Patentblatt 89/41**

(45) Bekanntmachung des Hinweises auf die Patenterteilung:
**07.11.90 Patentblatt 90/45**

(84) Benannte Vertragsstaaten:
**AT BE CH DE ES FR GB GR IT LI NL SE**

(56) Entgegenhaltungen:
**EP-A- 0 203 608**
**EP-A- 0 226 917**
**GB-A- 2 202 844**

(73) Patentinhaber: **BASF Aktiengesellschaft,**
**Carl-Bosch-Strasse 38, D-6700 Ludwigshafen(DE)**

(72) Erfinder: **Schuetz, Franz, Dr., Budapester Strasse 45, D-6700 Ludwigshafen(DE)**
Erfinder: **Brand, Siegbert, Dr., Hegelstrasse 39, D-6940 Weinheim(DE)**
Erfinder: **Wenderoth, Bernd, Dr., Schwalbenstrasse 26, D-6840 Lampertheim(DE)**
Erfinder: **Sauter, Hubert, Dr., Neckarpromenade 20, D-6800 Mannheim 1(DE)**
Erfinder: **Ammermann, Eberhard, Dr., Sachsenstrasse 3, D-6700 Ludwigshafen(DE)**
Erfinder: **Lorenz, Gisela, Dr., Erlenweg 13, D-6730 Neustadt(DE)**

EP 0 336 211 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft wertvolle neue ortho-substituierte Phenolether mit fungizider Wirkung und Fungizide, die diese Verbindungen enthalten.

Es ist bekannt, Acrylsäuremethylester, z.B. den α-(2-Benzyloxyphenyl)-β-methoxyacrylsäuremethylester (DE-35 19 282.8) oder den α-(2-Phenoxymethylenphenyl)-β-methoxyacrylsäuremethylester (DE-35 45 319.2) als Fungizide zu verwenden. Ihre fungizide Wirkung ist jedoch unbefriedigend.

Es wurde gefunden, daß ortho-substituierte Phenolether der allgemeinen Formel I

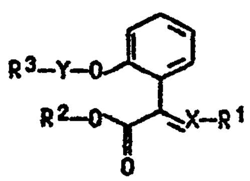

I,

in der

$R^1$ $C_1$-$C_4$-Alkyl, $C_1$-$C_4$-Alkoxy, $C_1$-$C_4$-Alkylthio oder gegebenenfalls durch $C_1$-$C_4$-Alkyl einfach oder doppelt substituiertes Amino bedeutet,

$R^2$ $C_1$-$C_4$-Alkyl bedeutet,

$R^3$ Aryloxy, Arylthio oder Arylalkoxy bedeutet, wobei der aromatische Ring gegebenenfalls durch einen bis drei der folgenden Reste substituiert ist: Halogen, $C_1$-$C_6$-Alkyl, $C_1$-$C_6$-Alkoxy, $C_1$-$C_4$-Alkylthio, $C_1$-$C_2$-Halogenalkyl, Aryl, Aryl-$C_1$-$C_2$-alkoxy, $C_1$-$C_4$-Alkylcarbonyl, durch $C_1$-$C_4$-Alkyl einfach oder doppelt substituiertes Amino, Cyano, Nitro,

X CH oder N bedeutet,

Y einen gegebenenfalls ungesättigten $C_2$-$C_{12}$-Alkylenrest bedeutet,

eine ausgezeichnete fungizide Wirkung besitzen, die besser ist als die bekannter Acrylsäuremethylester.

Die in der allgemeinen Formel aufgeführten Reste können beispielsweise folgende Bedeutung haben:

$R^1$ kann z.B. gegebenenfalls verzweigtes $C_1$-$C_4$-Alkyl (z.B. Methyl, Ethyl, n-oder iso-Propyl, n- iso-, sec.- oder tert.-Butyl), $C_1$-$C_4$-Alkoxy (z.B. Methoxy, Ethoxy, n- oder iso-Propoxy, n-, iso-, sec.- oder tert.-Butoxy), $C_1$-$C_4$-Alkylthio (z.B. Methylthio, Ethylthio, n- oder iso-Propylthio, n-, iso-, sec.- oder tert.-Butylthio), gegebenenfalls durch $C_1$-$C_4$-Alkyl einfach oder doppelt substituiertes Amino (z.B. Amino, Methylamino, Dimethylamino, Diethylamino, Diisopropylamino) sein.

$R^2$ kann z.B. $C_1$-$C_4$-Alkyl (z.B. Methyl, Ethyl, n- oder iso-Propyl, n-, iso-, sec.- oder tert.-Butyl) sein.

$R^3$ kann z.B. Aryloxy (Phenoxy, 1-Naphthyloxy, 2-Naphthyloxy), Arylthio (Phenylthio) oder Arylalkoxy (Benzyloxy) sein, wobei der aromatische Ring gegebenenfalls durch einen bis drei der folgenden Reste substituiert sein kann:

Halogen, (z.B. Fluor, Chlor, Brom), $C_1$-$C_6$-Alkyl (z.B. Methyl, Ethyl, n-oder iso-Propyl, n-, iso-, sec.- oder tert.-Butyl, n-, iso-, sec.-, tert.-oder neo-Pentyl, Hexyl), $C_1$-$C_6$-Alkoxy (z.B. Methoxy, Ethoxy, n- oder iso-Propoxy, n-, iso-, sec.- oder tert.-Butoxy), $C_1$-$C_4$-Alkylthio (z.B. Methylthio, Ethylthio), $C_1$-$C_2$-Halogenalkyl (z.B. Difluormethyl, Trifluormethyl), Aryl (z.B. Phenyl), Aryl-$C_1$-$C_2$-alkoxy (z.B. Benzyloxy), $C_1$-$C_4$-Alkylcarbonyl (z.B. Acetyl), durch $C_1$-$C_4$-Alkyl einfach oder doppelt substuiertes Amino (z.B. Dimethylamino), Cyano, Nitro.

Der in der allgemeinen Formel I aufgeführte Rest X kann CH oder N bedeuten,

Y kann z.B. bedeuten: einen geradkettigen $C_2$-$C_{12}$-Alkylenrest (z.B. Ethylen, Propylen, Butylen, Pentylen, Hexylen, Heptylen, Octylen, Nonylen, Decylen), einen verzweigten $C_2$-$C_{12}$-Alkylenrest (z.B. Methylethylen), einen $C_4$-$C_{12}$-Alkenylenrest (z.B. Butenylen).

Die neuen Verbindungen können beispielsweise nach folgenden Verfahren hergestellt werden:

Die Darstellung der Verbindungen der allgemeinen Formel Ia ($R^1$ = Alkoxy, X = CH, $R^2$, $R^3$ und Y haben die oben angegebene Bedeutung) erfolgt z.B. durch Umsetzung der ortho-substituierten Phenylessigester der allgemeinen Formel III mit Ameisensäuremethylester unter Verwendung einer Base (z.B. Natriumhydrid) in einem inerten Lösungmittel wie z.B. Diethylether oder Tetrahydrofuran (vgl. dazu Ann. Chem. 424 (1921) 214). Die so erhaltenen Hydroxymethylenderivate der allgemeinen Formel IV, die auch im Gleichgewicht mit den Formylderivaten V vorliegen können, werden mit einem Alkylierungsmittel (z.B. Dimethylsulfat) in Gegenwart einer Base (z.B. Kaliumcarbonat) in einem Verdünnungsmittel (z.B Aceton) zur Reaktion gebracht. In den folgenden Formelbildern bedeutet "Alk" eine $C_1$-$C_4$-Alkylgruppe und L eine Abgangsgruppe (z.B. Methylsulfat).

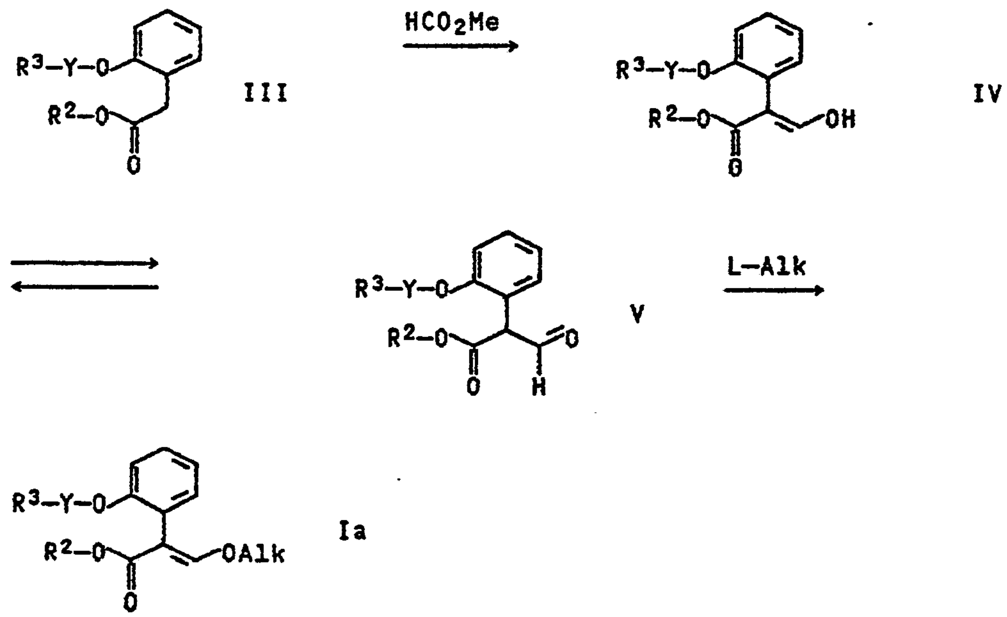


III IV V Ia

Zur Darstellung der Verbindungen der allgemeinen Formel Ib ($R^1$ = Alkylthio, X = CH, $R^2$, $R^3$ und Y haben die oben angegebene Bedeutung) werden die Hydroxymethylenderivate IV, die auch im Gleichgewicht mit den Formylderivaten V vorliegen können, zunächst mit Sulfonsäurechloriden wie z.B. Methansulfochlorid (R′ = Methyl), Trifluormethansulfochlorid (R′ = Trifluormethyl) oder p-Toluolsulfonsäurechlorid (R′ = p-Methyl-phenyl) in Gegenwart von Basen (z.B. Triethylamin) zu Verbindungen der allgemeinen Formel VI umgesetzt. Anschließend erhält man die gewünschten Verbindungen Ib durch Umsetzung von VI mit Alkylthiolaten Alk $S^-$, wie z.B. Natriumthiomethylat.

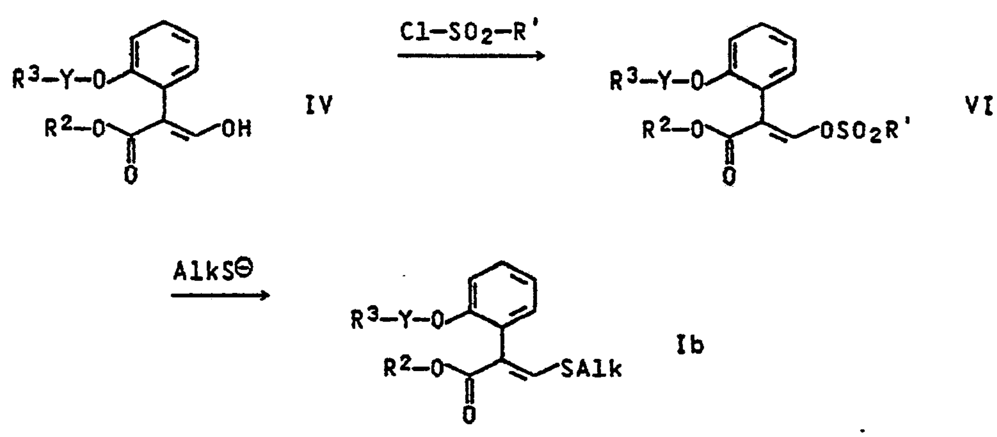


IV VI Ib

Die Darstellung der Verbindungen der allgemeinen Formel Ic ($R^1$ = Alkylamino bzw. Dialkylamino, X = CH, $R^2$, $R^3$ und Y haben die oben angegebene Bedeutung) erfolgt durch Umsetzung der Hydroxymethylenderivate IV, die auch im Gleichgewicht mit V vorliegen können, mit primären oder sekundären Aminen. Alternativ können auch die Alkalisalze von IV mit den Hydrochloriden von primären oder sekundären Aminen unter Freisetzung von Natriumchlorid umgesetzt werden (vgl. dazu Ann. Chim. [10] 18 (1932) 103).

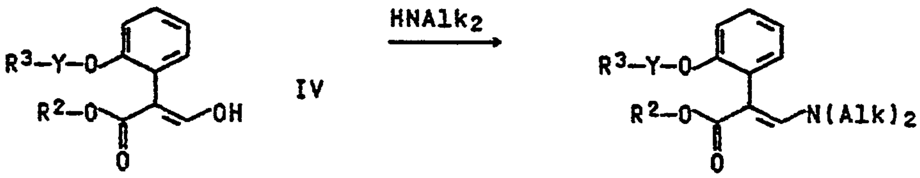


IV   Ic

Zu Verbindungen der Formel Ic kommt man auch, wenn man die ortho-substituierten Phenylessigester der allgemeinen Formel III mit Dialkylformamiddialkylacetalen oder mit Aminal-alkylestern gegebenenfalls unter p-Toluolsulfonsäure-Katalyse umsetzt (vgl. z.B. Chem. Ber. 97 (1964) 3396).

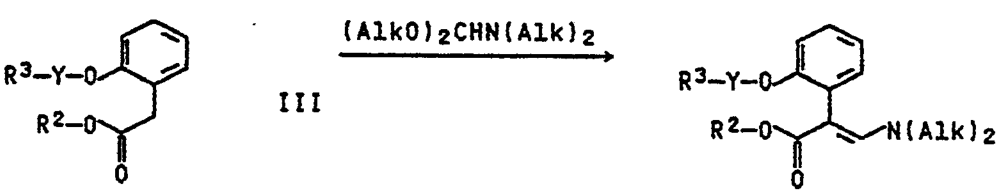


III   Ic

Die neuen Verbindungen der allgemeinen Formel Id ($R^1$ = Alkyl, X = CH, $R^2$, $R^3$ und Y haben die oben angegebene Bedeutung) lassen sich herstellen, indem man einen alpha-Keto-carbonsäureester der allgemeinen Formel II in einer Wittig-Reaktion mit einem Alkyltriphenylphosphoniumbromid in Gegenwart einer Base (z.B. n-Butyllithium, Natriummethylat, Kalium-tert.-butylat oder Natriumhydrid) in einem inerten Lösungsmittel (z.B. Diethylether, Tetrahydrofuran, Dimethylformamid, Dimethylsulfoxid) umsetzt (vgl. dazu Methoden der organischen Chemie, Band E1, S. 710, Thieme, Stuttgart 1982).

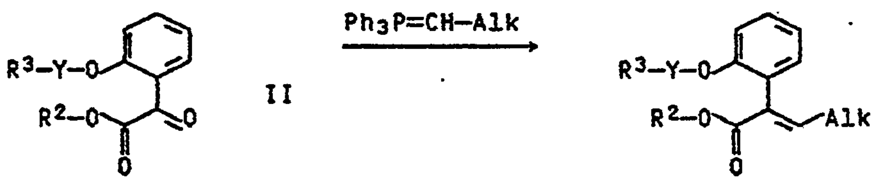


II   Id

Die alpha-Ketocarbonsäureester der allgemeinen Formel II sind neu. Sie können z.B. durch Umsetzung der entsprechenden aromatischen Grignard Verbindung VII mit Imidazoliden der Formel VIII hergestellt werden (J. Org. Chem. 46 (1981) 211). $R^2$, $R^3$ und Y haben die oben angegebene Bedeutung.

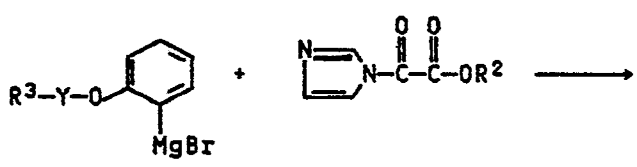

II

VII   VIII

Die neuen Verbindungen der allgemeinen Formel Ie ($R^1$ = Alkoxy, X = N, $R^2$, $R^3$ und Y haben die oben angegebene Bedeutung) werden dadurch gewonnen, daß man die alpha-Ketocarbonsäureester II mit einem 0-Alkyl-substituierten Hydroxylamin-hydrochlorid in Gegenwart einer Base (z.B. Natriumcarbonat, Natriumacetat) in einem inerten Lösungsmittel (z.B. Methanol) umsetzt. Die Verbindungen der Formel Ie erhält man auch, wenn man die alpha-Ketocarbonsäureester II zunächst mit Hydroxylamin umsetzt und die resultierenden Oxime anschließend mit Alkylierungmitteln der Formel Alk-L (L = Halogen, z.B. Cl, Br, J) zur Reaktion bringt.

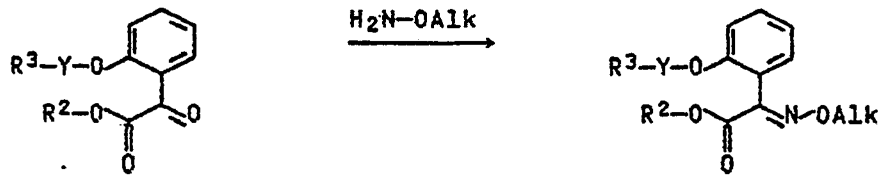


Ie

Ein Herstellungsverfahren für ortho-substituierte Phenolether der allgemeinen Formel If ($R^1$ = Alkylamino bzw. Dialkylamino, X = N, $R^2$, $R^3$ und Y haben die oben angegebene Bedeutung) ist z.B. das folgende: alpha-Ketocarbonsäureester der Formel II werden mit substituierten Hydrazinen in Gegenwart einer Protonsäure (z.B. Salzsäure) in einem geeigneten Lösungsmittel (z.B. Methanol) umgesetzt (vgl. dazu Liebigs Ann. Chem. 722 (1969) 29).

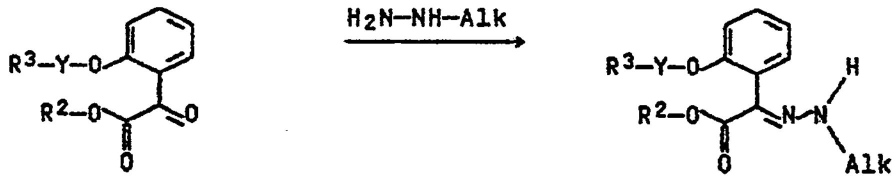


If

Die Herstellung der als Ausgangsverbindungen benötigten ortho-substituierten Phenylessigester III erfolgt z.B. durch Alkylierung von ortho-Hydroxy-phenylessigsäuremethylester

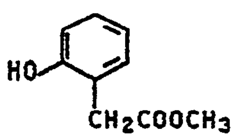

mit omega-Aryloxyalkylhalogeniden
$R^3$-Y-Halogen
nach an sich bekannten Verfahren (Methoden der organischen Chemie, Band 6/3, S. 54, Thieme, Stuttgart 1965). Auf die gleiche Weise erhält man aus ortho-Hydroxyphenylbromid

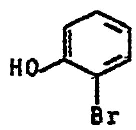

und omega-Aryloxyalkylhalgeniden
$R^3$-Y-Halogen
ortho-substituierte Phenylbromide

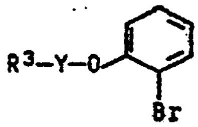

die nach Standardmethoden zu den als Ausgangsverbindungen benötigten Grignard-Verbindungen VII weiter umgesetzt werden.

Omega-Aryloxyalkylhalogenide $R^3$-Y-Halogen sind bekannte Verbindungen oder können leicht nach bekannten Vefahren hergestellt werden, z.B. durch Monoalkylierung von Phenolen mit aliphatischen Dihalogenalkanen (z.B. 1,2-Dibrommethan, 1,3-Dibrompropan, 1,4-Dibrombutan, 1,5-Dibrompropan, 1;6-Dibromhexan, 1,7-Dibromheptan).

Die neuen Verbindungen der Formel I fallen bei der Herstellung aufgrund der C=C- bzw. C=N-Doppelbindung zum Teil als E/Z-Isomerengemische an. Diese können in üblicher Weise, z.B. durch Kristallisation oder Chromatographie, in die einzelnen Komponenten getrennt werden. Sowohl die einzelnen isomeren Verbindungen als auch ihre Gemische werden von der Erfindung umfaßt.

Das folgende Beispiel soll die Herstellung der neuen Wirkstoffe erläutern.

Vorschrift 1

2-(4-Phenoxy-butyl-1-oxy)-phenylessigsäuremethylester

28,2 g (0,17 mol) 2-Hydroxyphenylessigsäuremethylester, 25,8 g (0,19 mol) Kaliumcarbonat und 38,9 g (0,17 mol) 4-Phenoxybutylbromid werden 24 Stunden in 400 ml Dimethylformamid bei 70°C gerührt. Anschließend wird das Lösungsmittel abgezogen und der Rückstand in Diethylether aufgenommen. Man extrahiert mehrmals mit Wasser und gesättigter Natriumcarbonatlösung und trocknet die organische Phase über $MgSO_4$. Das nach dem Entfernen des Lösungsmittels erhaltene Öl wird durch Chromatographie an Kieselgel (Cyclohexan/Essigester) gereinigt. Ausbeute: 31 g (58 %).

Vorschrift 2

alpha-[2-(4-phenoxy-butyl-1-oxy)-phenyl]-β-hydroxyacrylsäuremethylester

Zu einer Suspension von 3,4 g (0,14 mol) Natriumhydrid in 50 ml Diethylether tropft man bei Raumtemperatur eine Mischung aus 30 g (0,10 mol) 2-(4-Phenoxy-butyl-1-oxy)-phenylessigsäuremethylester, 12,5 g (0,21 mol) Ameisensäuremethylester und 100 ml Diethylether. Man rührt 12 Stunden bei Raumtemperatur und hydrolysiert anschließend durch Zugabe von Eis. Die wäßrige, alkalische Phase wird mit Diethylether extrahiert, mit verd. HCl angesäuert und erneut mit Diethylether ausgeschüttelt. Nach dem Einengen der organischen Phase erhält man 21 g (71 %) alpha-2-(4-Phenoxy-butyl-1-oxy)-phenyl]-β-hydroxyacrylsäure -methylester.

Beispiel 1

alpha-2-(4-Phenoxy-butyl-1-oxy)-phenyl]-β-methoxyacrylsäure-methylester

20 g (0,06 mol) der oben erhaltenen Verbindung werden zusammen mit 7,4 g (0,06 mol) Dimethylsulfat und 8,0 g (0,06 mol) Kaliumcarbonat 48 Stunden in 150 ml Aceton bei Raumtemperatur gerührt. Anschließend wird vom Niederschlag abfiltriert, das Aceton abgezogen und der Rückstand in Diethylether aufgenommen. Die organische Phase wird mit halbkonzentriertem Ammoniak und Wasser gewaschen, getrocknet und eingeengt. Man erhält 15,2 g (73 %) der Titelverbindung als reines E-Isomeres in Form farbloser Kristalle vom Schmelzpunkt 88°C (Verbindung Nr. 97).

In entsprechender Weise lassen sich die folgenden Verbindungen herstellen:

Tabelle 1:

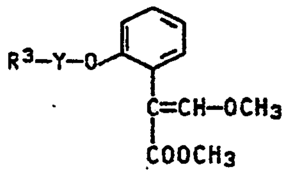

Verbindungen der Formel I
($R^1 = OCH_3$, $R^2 = CH_3$, X = CH)
Die Konfigurationsangabe bezieht sich auf die ß-Methoxy-acrylestergruppe

| Nr. | $R^3$ | Y | Fp. (Isomeres) |
|---|---|---|---|
| 1 | $C_6H_5$-O- (= Phenoxy) | $-CH_2-CH_2-$ | |
| 2 | 2-F-$C_6H_4$-O- | $-CH_2-CH_2-$ | |
| 3 | 3-F-$C_6H_4$-O- | $-CH_2-CH_2-$ | |
| 4 | 4-F-$C_6H_4$-O- | $-CH_2-CH_2-$ | |
| 5 | 2-Cl-$C_6H_4$-O- | $-CH_2-CH_2-$ | |
| 6 | 3-Cl-$C_6H_4$-O- | $-CH_2-CH_2-$ | |
| 7 | 4-Cl-$C_6H_4$-O- | $-CH_2-CH_2-$ | |
| 8 | 2-$CH_3$-$C_6H_4$-O- | $-CH_2-CH_2-$ | |
| 9 | 3-$CH_3$-$C_6H_4$-O- | $-CH_2-CH_2-$ | |
| 10 | 4-$CH_3$-$C_6H_4$-O- | $-CH_2-CH_2-$ | |
| 11 | 2-$OCH_3$-$C_6H_4$-O- | $-CH_2-CH_2-$ | |
| 12 | 3-$OCH_3$-$C_6H_4$-O- | $-CH_2-CH_2-$ | |
| 13 | 4-$OCH_3$-$C_6H_4$-O- | $-CH_2-CH_2-$ | |
| 14 | 4-t-$C_4H_9$-$C_6H_4$-O- | $-CH_2-CH_2-$ | |
| 15 | 4-$OC_2H_5$-$C_6H_4$-O- | $-CH_2-CH_2-$ | |
| 16 | 4-$CF_3$-$C_6H_4$-O- | $-CH_2-CH_2-$ | |
| 17 | 4-$C_6H_5$-$C_6H_4$-O- | $-CH_2-CH_2-$ | |
| 18 | 4-($C_6H_5$-$CH_2$-O-)-$C_6H_4$-O- | $-CH_2-CH_2-$ | |
| 19 | 2,4,6-$Cl_3$-$C_6H_2$-O- | $-CH_2-CH_2-$ | |
| 20 | 2,4,6-$(CH_3)_3$-$C_6H_2$-O- | $-CH_2-CH_2$ | |
| 21 | 1-Naphthol | $-CH_2-CH_2-$ | |
| 22 | 2-Naphthol | $-CH_2-CH_2-$ | |
| 23 | 2-Br-$C_6H_4$-O- | $-CH_2-CH_2-$ | |
| 24 | 3-Br-$C_6H_4$-O- | $-CH_2-CH_2-$ | |
| 25 | 4-Br-$C_6H_4$-O- | $-CH_2-CH_2-$ | |
| 26 | 2-$CF_3$-$C_6H_4$-O- | $-CH_2-CH_2-$ | |
| 27 | 3-$CF_3$-$C_6H_4$-O- | $-CH_2-CH_2-$ | |
| 28 | 4-CN-$C_6H_4$-O- | $-CH_2-CH_2-$ | |
| 29 | 4-$CH_3CO$-$C_6H_4$-O- | $-CH_2-CH_2-$ | |

Tabelle 1 (Fortsetzung

| Nr. | $R^3$ | Y | Fp. (Isomeres) |
|---|---|---|---|
| 30 | $3-(CH_3)_2N-C_6H_4-O-$ | $-CH_2-CH_2-$ | |
| 31 | $4-SCH_3-C_6H_4-O-$ | $-CH_2-CH_2-$ | |
| 32 | $2-OC_2H_5-C_6H_4-O-$ | $-CH_2-CH_2-$ | |
| 33 | $4-t-Butoxy-C_6H_4-O-$ | $-CH_2-CH_2-$ | |
| 34 | $2,4-Cl_2-C_6H_3-O-$ | $-CH_2-CH_2-$ | |
| 35 | $3,4-Cl_2-C_6H_3-O-$ | $-CH_2-CH_2-$ | |
| 36 | $2,5-Cl_2-C_6H_3-O-$ | $-CH_2-CH_2-$ | |
| 37 | $2,6-Cl_2-C_6H_3-O-$ | $-CH_2-CH_2-$ | |
| 38 | $2,4,5-Cl_3-C_6H_2-O-$ | $-CH_2-CH_2-$ | |
| 39 | $2-Cl-4-F-C_6H_3-O-$ | $-CH_2-CH_2-$ | |
| 40 | $2-Cl-4-Br-C_6H_3-O-$ | $-CH_2-CH_2-$ | |
| 41 | $2,4-(CH_3)_2-C_6H_3-O-$ | $-CH_2-CH_2-$ | |
| 42 | $2-CH_3-4-t-C_4H_9-C_6H_3-O-$ | $-CH_2-CH_2-$ | |
| 43 | $2-CH_3-4-Cl-C_6H_3-O-$ | $-CH_2-CH_2-$ | |
| 44 | $2-CH_3-4,6-Cl_2-C_6H_2-O-$ | $-CH_2-CH_2-$ | |
| 45 | $2,6-(CH_3)_2-C_6H_3-O-$ | $-CH_2-CH_2-$ | |
| 46 | $2,5,6-(CH_3)_3-C_6H_2-O-$ | $-CH_2-CH_2-$ | |
| 47 | $2,4,5-(CH_3)_3-C_6H_2-O-$ | $-CH_2-CH_2-$ | |
| 48 | $2-i-C_3H_7-5-CH_3-C_6H_3-O-$ | $-CH_2-CH_2-$ | |
| 49 | $3,5-(C_2H_5)_2-C_6H_3-O-$ | $-CH_2-CH_2-$ | |
| 50 | $2,4,6-(sec-Butyl)_3-C_6H_2-O-$ | $-CH_2-CH_2-$ | |
| 51 | $3,5-(OCH_3)_2-C_6H_3-O-$ | $-CH_2-CH_2-$ | |
| 52 | $2-OCH_3-4-CH_3-C_6H_3-O-$ | $-CH_2-CH_2-$ | |
| 53 | $2,6-(CH_3)_2-4-SCH_3-C_6H_2-O-$ | $-CH_2-CH_2-$ | |
| 54 | $2-Cl-5-OCH_3-C_6H_3-O-$ | $-CH_2-CH_2-$ | |
| 55 | $2-CH_3-4-SCH_3-C_6H_3-O-$ | $-CH_2-CH_2-$ | |
| 56 | $2,6-(OCH_3)_2-C_6H_3-O-$ | $-CH_2-CH_2-$ | |
| 57 | $2-Cl-4-C_6H_5-C_6H_3-O-$ | $-CH_2-CH_2$ | |
| 58 | $2-Cl-4-CH_3-C_6H_3-O-$ | $-CH_2-CH_2-$ | |
| 59 | $2-CH_3-6-i-C_3H_7-C_6H_3-O-$ | $-CH_2-CH_2-$ | |
| 60 | $3-t-C_4H_9-4-OCH_3-C_6H_3-O-$ | $-CH_2-CH_2-$ | |
| 61 | $2,4-(CH_3)_2-1-Naphthol$ | $-CH_2-CH_2-$ | |
| 62 | $C_6H_5-S-$ | $-CH_2-CH_2-$ | |
| 63 | $4-Cl-C_6H_4-S-$ | $-CH_2-CH_2-$ | |
| 64 | $2,4,6-Cl_3-C_6H_2-O-$ | $-CH_2-CH(CH_3)-$ | |
| 65 | $C_6H_5-O-$ | $-CH_2-CH(CH_3)-$ | |
| 66 | $C_6H_5-O-$ | $-(CH_2)_3-$ | Oel (E) |
| 67 | $2-F-C_6H_4-O-$ | $-(CH_2)_3-$ | |
| 68 | $3-F-C_6H_4-O-$ | $-(CH_2)_3-$ | |
| 69 | $4-F-C_6H_4-O-$ | $-(CH_2)_3-$ | |

Tabelle 1 (Fortsetzung)

| Nr. | $R^3$ | Y | Fp. (Isomeres) |
|---|---|---|---|
| 70 | $2-Cl-C_6H_4-O-$ | $-(CH_2)_3-$ | |
| 71 | $3-Cl-C_6H_4-O-$ | $-(CH_2)_3-$ | |
| 72 | $4-Cl-C_6H_4-O-$ | $-(CH_2)_3-$ | |
| 73 | $2-CH_3-C_6H_4-O-$ | $-(CH_2)_3-$ | |
| 74 | $3-CH_3-C_6H_4-O-$ | $-(CH_2)_3-$ | |
| 75 | $4-CH_3-C_6H_4-O-$ | $-(CH_2)_3-$ | |
| 76 | $2-OCH_3-C_6H_4-O-$ | $-(CH_2)_3-$ | |
| 77 | $3-OCH_3-C_6H_4-O-$ | $-(CH_2)_3-$ | |
| 78 | $4-OCH_3-C_6H_4-O-$ | $-(CH_2)_3-$ | |
| 79 | $4-t-C_4H_9-C_6H_4-O-$ | $-(CH_2)_3-$ | |
| 80 | $4-OC_2H_5-C_6H_4-O-$ | $-(CH_2)_3-$ | |
| 81 | $4-CF_3-C_6H_4-O-$ | $-(CH_2)_3-$ | |
| 82 | $4-C_6H_5-C_6H_4-O-$ | $-(CH_2)_3-$ | |
| 83 | $4-(C_6H_5-CH_2-O)-C_6H_4-O-$ | $-(CH_2)_3-$ | |
| 84 | $2,4,6-Cl_3-C_6H_2-O-$ | $-(CH_2)_3-$ | |
| 85 | $2,4,6-(CH_3)_3-C_6H_2-O-$ | $-(CH_2)_3-$ | |
| 86 | 1-Naphthol | $-(CH_2)_3-$ | |
| 87 | 2-Naphthol | $-(CH_2)_3-$ | |
| 88 | $2,4-Cl_2-C_6H_3-O-$ | $-(CH_2)_3-$ | |
| 89 | $3,5-Cl_2-C_6H_3-O-$ | $-(CH_2)_3-$ | |
| 90 | $3,4-Cl_2-C_6H_3-O-$ | $-(CH_2)_3-$ | |
| 91 | $2,6-(CH_3)_2-C_6H_3-O-$ | $-(CH_2)_3-$ | |
| 92 | $2,4-(CH_3)_2-C_6H_3-O-$ | $-(CH_2)_3-$ | |
| 93 | $3-CF_3-C_6H_4-O-$ | $-(CH_2)_3-$ | |
| 94 | $2-Cl-4-CH_3-C_6H_3-O-$ | $-(CH_2)_3-$ | |
| 95 | $C_6H_5-S-$ | $-(CH_2)_3-$ | |
| 96 | $C_6H_5-CH_2-O-$ | $-(CH_2)_3-$ | |
| 97 | $C_6H_5-O-$ | $-(CH_2)_4-$ | 88°C (E) |
| 98 | $2-F-C_6H_4-O-$ | $-(CH_2)_4-$ | |
| 99 | $3-F-C_6H_4-O-$ | $-(CH_2)_4-$ | |
| 100 | $4-F-C_6H_4-O-$ | $-(CH_2)_4-$ | |
| 101 | $2-CH_3-C_6H_4-O-$ | $-(CH_2)_4-$ | |
| 102 | $3-CH_3-C_6H_4-O-$ | $-(CH_2)_4-$ | |
| 103 | $4-CH_3-C_6H_4-O-$ | $-(CH_2)_4-$ | |
| 104 | $2-OCH_3-C_6H_4-O-$ | $-(CH_2)_4-$ | |
| 105 | $3-OCH_3-C_6H_4-O-$ | $-(CH_2)_4-$ | |
| 106 | $4-OCH_3-C_6H_4-O-$ | $-(CH_2)_4-$ | |
| 107 | $4-t-C_4H_9-C_6H_4-O-$ | $-(CH_2)_4-$ | |
| 108 | $4-OC_2H_5-C_6H_4-O-$ | $-(CH_2)_4-$ | |

Tabelle 1 (Fortsetzung)

| Nr. | $R^3$ | Y | Fp. (Isomeres) |
|---|---|---|---|
| 109 | $4-CF_3-C_6H_4-O-$ | $-(CH_2)_4-$ | |
| 110 | $4-C_6H_5-C_6H_4-O-$ | $-(CH_2)_4$ | |
| 111 | $4-(C_6H_5-CH_2-O)-C_6H_4-$ | $-(CH_2)_4-$ | |
| 112 | $2,4,6-Cl_3-C_6H_2-O-$ | $-(CH_2)_4-$ | |
| 113 | $2,4,6-(CH_3)-C_6H_2-O-$ | $-(CH_2)_4-$ | |
| 114 | 1-Naphthol | $-(CH_2)_4-$ | |
| 115 | 2-Naphthol | $-(CH_2)_4-$ | |
| 116 | $3-CF_3-C_6H_4-O-$ | $-(CH_2)_4-$ | |
| 117 | $3-iso-C_3H_7-C_6H_4-O-$ | $-(CH_2)_4-$ | |
| 118 | $4-iso-C_3H_7-C_6H_4-O-$ | $-(CH_2)_4-$ | |
| 119 | $3-t-C_4H_9-C_6H_4-O-$ | $-(CH_2)_4-$ | |
| 120 | $4-t-Butoxy-C_6H_4-O-$ | $-(CH_2)_4-$ | |
| 121 | $3-C_6H_5-C_6H_4-O-$ | $-(CH_2)_4-$ | |
| 122 | $2,4-Cl_2-C_6H_3-O-$ | $-(CH_2)_4-$ | |
| 123 | $3,4-Cl_2-C_6H_3-O-$ | $-(CH_2)_4-$ | |
| 124 | $3,5-Cl_2-C_6H_3-O-$ | $-(CH_2)_4-$ | |
| 125 | $2,6-Cl_2-C_6H_3-O-$ | $-(CH_2)_4-$ | |
| 126 | $2-Cl-4-CH_3-C_6H_3-O-$ | $-(CH_2)_4-$ | |
| 127 | $2,6-(CH_3)_2-C_6H_3-O-$ | $-(CH_2)_4-$ | |
| 128 | $2,5-(CH_3)_2-C_6H_3-O-$ | $-(CH_2)_4-$ | |
| 129 | $4-CN-C_6H_4-O-$ | $-(CH_2)_4-$ | |
| 130 | $C_6H_5-S-$ | $-(CH_2)_4-$ | |
| 131 | $C_6H_5-CH_2-O-$ | $-(CH_2)_4-$ | |
| 132 | $C_6H_5-O-$ | $-CH_2-CH=CH-CH_2-$ | |
| 133 | $2-F-C_6H_4-O-$ | $-CH_2-CH=CH-CH_2-$ | |
| 134 | $3-F-C_6H_4-O-$ | $-CH_2-CH=CH-CH_2-$ | |
| 135 | $4-F-C_6H_4-O-$ | $-CH_2-CH=CH-CH_2-$ | |
| 136 | $2-Cl-C_6H_4-O-$ | $-CH_2-CH=CH-CH_2-$ | |
| 137 | $3-Cl-C_6H_4-O-$ | $-CH_2-CH=CH-CH_2-$ | |
| 138 | $4-Cl-C_6H_4-O-$ | $-CH_2-CH=CH-CH_2-$ | |
| 139 | $C_6H_5-O-$ | $-(CH_2)_5-$ | |
| 140 | $2-Cl-C_6H_4-O-$ | $-(CH_2)_5-$ | Oel (E) |
| 141 | $3-Cl-C_6H_4-O-$ | $-(CH_2)_5-$ | |
| 142 | $4-Cl-C_6H_4-O-$ | $-(CH_2)_5-$ | |
| 143 | $2-F-C_6H_4-O-$ | $-(CH_2)_5-$ | |
| 144 | $3-F-C_6H_4-O-$ | $-(CH_2)_5-$ | |
| 145 | $4-F-C_6H_4-O-$ | $-(CH_2)_5-$ | |
| 146 | $2-CH_3-C_6H_4-O-$ | $-(CH_2)_5-$ | |
| 147 | $3-CH_3-C_6H_4-O-$ | $-(CH_2)_5-$ | |

Tabelle 1 (Fortsetzung)

| Nr. | $R^3$ | Y | Fp. (Isomeres) |
|---|---|---|---|
| 148 | $4-CH_3-C_6H_4-O-$ | $-(CH_2)_5-$ | |
| 149 | $2-OCH_3-C_6H_4-O-$ | $-(CH_2)_5-$ | |
| 150 | $3-OCH_3-C_6H_4-O-$ | $-(CH_2)_5-$ | |
| 151 | $4-OCH_3-C_6H_4-O-$ | $-(CH_2)_5-$ | |
| 152 | $4-t-C_4H_9-C_6H_4-O-$ | $-(CH_2)_5-$ | |
| 153 | $4-OC_2H_5-C_6H_4-O-$ | $-(CH_2)_5-$ | |
| 154 | $4-CF_3-C_6H_4-O-$ | $-(CH_2)_5-$ | |
| 155 | $4-C_6H_5-C_6H_4-O-$ | $-(CH_2)_5-$ | |
| 156 | $4-(C_6H_5-CH_2-O)-C_6H_4-O-$ | $-(CH_2)_5-$ | |
| 157 | $2,4,6-Cl_3-C_6H_2-O-$ | $-(CH_2)_5-$ | |
| 158 | $2,4,6-(CH_3)_3-C_6H_2-O-$ | $-(CH_2)_5-$ | |
| 159 | 1-Naphthol | $-(CH_2)_5-$ | |
| 160 | 2-Naphthol | $-(CH_2)_5-$ | |
| 161 | $C_6H_5-CH_2-O-$ | $-(CH_2)_5-$ | |
| 162 | $C_6H_5-O-$ | $-(CH_2)_6-$ | 88°C (E) |
| 163 | $2-Cl-C_6H_4-O-$ | $-(CH_2)_6-$ | Öl (E) |
| 164 | $2-Cl-C_6H_4-O-$ | $-(CH_2)_6-$ | Öl (E/Z=3/1) |
| 165 | $3-Cl-C_6H_4-O-$ | $-(CH_2)_6-$ | |
| 166 | $4-Cl-C_6H_4-O-$ | $-(CH_2)_6-$ | 59°C (E) |
| 167 | $2-F-C_6H_4-O-$ | $-(CH_2)_6-$ | |
| 168 | $3-F-C_6H_4-O-$ | $-(CH_2)_6-$ | |
| 169 | $4-F-C_6H_4-O-$ | $-(CH_2)_6-$ | |
| 170 | $2-CH_3-C_6H_4-O-$ | $-(CH_2)_6-$ | |
| 171 | $3-CH_3-C_6H_4-O-$ | $-(CH_2)_6-$ | |
| 172 | $4-CH_3-C_6H_4-O-$ | $-(CH_2)_6-$ | |
| 173 | $2-OCH_3-C_6H_4-O-$ | $-(CH_2)_6-$ | |
| 174 | $3-OCH_3-C_6H_4-O-$ | $-(CH_2)_6-$ | |
| 175 | $4-OCH_3-C_6H_4-O-$ | $-(CH_2)_6-$ | |
| 176 | $4-t-C_4H_9-C_6H_4-O-$ | $-(CH_2)_6-$ | |
| 177 | $4-OC_2H_5-C_6H_4-O-$ | $-(CH_2)_6-$ | |
| 178 | $4-CF_3-C_6H_4-O-$ | $-(CH_2)_6-$ | |
| 179 | $4-C_6H_5-C_6H_4-O-$ | $-(CH_2)_6-$ | |
| 180 | $4-(C_6H_5-CH_2-O)-C_6H_4-O-$ | $-(CH_2)_6-$ | |
| 181 | $2,4,6-Cl_3-C_6H_2-O-$ | $-(CH_2)_6-$ | |
| 182 | $2,4,6-(CH_3)_3-C_6H_2-O-$ | $-(CH_2)_6-$ | |
| 183 | 1-Naphthol | $-(CH_2)_6-$ | |
| 184 | 2-Naphthol | $-(CH_2)_6-$ | |

Tabelle 1 (Fortsetzung)

| Nr. | $R^3$ | Y | Fp. (Isomeres) |
|---|---|---|---|
| 185 | $C_6H_5-CH_2-O-$ | $-(CH_2)_6-$ | |
| 186 | $C_6H_5-O-$ | $-(CH_2)_7-$ | |
| 187 | $2-Cl-C_6H_4-O-$ | $-(CH_2)_7-$ | |
| 188 | $3-Cl-C_6H_4-O-$ | $-(CH_2)_7-$ | |
| 189 | $4-Cl-C_6H_4-O-$ | $-(CH_2)_7-$ | |
| 190 | $2-F-C_6H_4-O-$ | $-(CH_2)_7-$ | |
| 191 | $3-F-C_6H_4-O-$ | $-(CH_2)_7-$ | |
| 192 | $4-F-C_6H_4-O-$ | $-(CH_2)_7-$ | |
| 193 | $2-CH_3-C_6H_4-O-$ | $-(CH_2)_7-$ | |
| 194 | $3-CH_3-C_6H_4-O-$ | $-(CH_2)_7-$ | |
| 195 | $4-CH_3-C_6H_4-O-$ | $-(CH_2)_7-$ | öl (E) |
| 196 | $2-OCH_3-C_6H_4-O-$ | $-(CH_2)_7-$ | |
| 197 | $3-OCH_3-C_6H_4-O-$ | $-(CH_2)_7-$ | |
| 198 | $4-OCH_3-C_6H_4-O-$ | $-(CH_2)_7-$ | |
| 199 | $4-t-C_4H_9-C_6H_4-O-$ | $-(CH_2)_7-$ | |
| 200 | $4-OC_2H_5-C_6H_4-O-$ | $-(CH_2)_7-$ | |
| 201 | $4-CF_3-C_6H_4-O-$ | $-(CH_2)_7-$ | |
| 202 | $4-C_6H_5-C_6H_4-O-$ | $-(CH_2)_7-$ | |
| 203 | $2,4,6-(Cl_3)_3-C_6H_2-O-$ | $-(CH_2)_7-$ | |
| 204 | $2,4,6-(CH_3-C_6H_2-O-$ | $-(CH_2)_7-$ | |
| 205 | 1-Naphthol | $-(CH_2)_7-$ | |
| 206 | 2-Naphthol | $-(CH_2)_7-$ | |
| 207 | $4-(C_6H_5-CH_2-O)-C_6H_4-O-$ | $-(CH_2)_7-$ | |
| 208 | $C_6H_5-CH_2-O-$ | $-(CH_2)_7-$ | |
| 209 | $C_6H_5-O-$ | $-(CH_2)_8-$ | öl (E) |
| 210 | $2-Cl-C_6H_4-O-$ | $-(CH_2)_8-$ | |
| 211 | $3-Cl-C_6H_4-O-$ | $-(CH_2)_8-$ | |
| 212 | $4-Cl-C_6H_4-O-$ | $-(CH_2)_8-$ | |
| 213 | $2-F-C_6H_4-O-$ | $-(CH_2)_8-$ | |
| 214 | $3-F-C_6H_4-O-$ | $-(CH_2)_8-$ | |
| 215 | $4-F-C_6H_4-O-$ | $-(CH_2)_8-$ | |
| 216 | $2-CH_3-C_6H_4-O-$ | $-(CH_2)_8-$ | |
| 217 | $3-CH_3-C_6H_4-O-$ | $-(CH_2)_8-$ | |
| 218 | $4-CH_3-C_6H_4-O-$ | $-(CH_2)_8-$ | |
| 219 | $2-OCH_3-C_6H_4-O-$ | $-(CH_2)_8-$ | |
| 220 | $3-OCH_3-C_6H_4-O-$ | $-(CH_2)_8-$ | |
| 221 | $4-OCH_3-C_6H_4-O-$ | $-(CH_2)_8-$ | |
| 222 | $4-t-C_4H_9-C_6H_4-O-$ | $-(CH_2)_8-$ | |
| 223 | $4-OC_2H_5-C_6H_4-O-$ | $-(CH_2)_8-$ | |

Tabelle 1 (Fortsetzung)

| Nr. | $R^3$ | Y | Fp. (Isomeres) |
|---|---|---|---|
| 224 | $4-CF_3-C_6H_4-O-$ | $-(CH_2)_8-$ | |
| 225 | $4-C_6H_5-C_6H_4-O-$ | $-(CH_2)_8-$ | |
| 226 | $4-(C_6H_5-CH_2-O)-C_6H_4-O-$ | $-(CH_2)_8-$ | |
| 227 | $2,4,6-Cl_3-C_6H_2-O-$ | $-(CH_2)_8-$ | |
| 228 | $2,4,6-(CH_3)_3-C_6H_2-O-$ | $-(CH_2)_8-$ | |
| 229 | 1-Naphthol | $-(CH_2)_8-$ | |
| 230 | 2-Naphthol | $-(CH_2)_8-$ | |
| 231 | $C_6H_5-CH_2-O-$ | $-(CH_2)_8-$ | |
| 232 | $C_6H_5-O-$ | $-(CH_2)_9-$ | Öl (E) |
| 233 | $C_6H_5-O-$ | $-(CH_2)_{10}-$ | 56°C (E) |
| 234 | $2-Cl-C_6H_4-O-$ | $-(CH_2)_{10}-$ | |
| 235 | $3-Cl-C_6H_4-O-$ | $-(CH_2)_{10}-$ | |
| 236 | $4-Cl-C_6H_4-O-$ | $-(CH_2)_{10}-$ | |
| 237 | $2-F-C_6H_4-O-$ | $-(CH_2)_{10}-$ | |
| 238 | $3-F-C_6H_4-O-$ | $-(CH_2)_{10}-$ | |
| 239 | $4-F-C_6H_4-O-$ | $-(CH_2)_{10}-$ | |
| 240 | $2-CH_3-C_6H_4-O-$ | $-(CH_2)_{10}-$ | |
| 241 | $3-CH_3-C_6H_4-O-$ | $-(CH_2)_{10}-$ | |
| 242 | $4-CH_3-C_6H_4-O-$ | $-(CH_2)_{10}-$ | |
| 243 | $2-OCH_3-C_6H_4-O-$ | $-(CH_2)_{10}-$ | |
| 244 | $3-OCH_3-C_6H_4-O-$ | $-(CH_2)_{10}-$ | |
| 245 | $4-OCH_3-C_6H_4-O-$ | $-(CH_2)_{10}-$ | |
| 246 | $4-t-C_4H_9-C_6H_4-O-$ | $-(CH_2)_{10}-$ | |
| 247 | $4-OC_2H_5-C_6H_4-O-$ | $-(CH_2)_{10}-$ | |
| 248 | $4-CF_3-C_6H_4-O-$ | $-(CH_2)_{10}-$ | |
| 249 | $4-C_6H_5-C_6H_4-O-$ | $-(CH_2)_{10}-$ | |
| 250 | $4-(C_6H_5-CH_2-O)-C_6H_4-O-$ | $-(CH_2)_{10}-$ | |
| 251 | $2,4,6-Cl_3-C_6H_2-O-$ | $-(CH_2)_{10}-$ | |
| 252 | $2,4,6-(CH_3)_3-C_6H_2-O-$ | $-(CH_2)_{10}-$ | |
| 253 | 1-Naphthol | $-(CH_2)_{10}-$ | |
| 254 | 2-Naphthol | $-(CH_2)_{10}-$ | |
| 255 | $C_6H_5-CH_2-O-$ | $-(CH_2)_{10}-$ | |
| 280 | $2,4-Cl_2-C_6H_3-O-$ | $-(CH_2)_6-$ | Öl (E) |
| 281 | $2,6-Cl_2-C_6H_3-O-$ | $-(CH_2)_6-$ | Öl (E) |
| 282 | $4-Cl-C_6H_4-O-$ | $-(CH_2)_{12}-$ | 61°C (E) |

Tabelle 2:

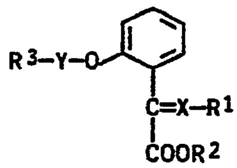

Verbindungen der Formel I

| Nr. | $R^1$ | $R^2$ | $R^3$ | X | Y | Fp. (Isomeres) |
|---|---|---|---|---|---|---|
| 256 | $CH_3$ | $CH_3$ | $C_6H_5$-O | CH | $(CH_2)_4$ | |
| 257 | $CH_3$ | $CH_3$ | 2-Cl-$C_6H_4$-O- | CH | $(CH_2)_5$ | |
| 258 | $CH_3$ | $CH_3$ | 2-Cl-$C_6H_4$-O- | CH | $(CH_2)_6$ | |
| 259 | $C_2H_5$ | $CH_3$ | $C_6H_5$-O- | CH | $(CH_2)_4$ | |
| 260 | $C_2H_5$ | $CH_3$ | 2-Cl-$C_6H_4$-O- | CH | $(CH_2)_5$ | |
| 261 | $C_2H_5$ | $CH_3$ | 2-Cl-$C_6H_4$-O- | CH | $(CH_2)_6$ | |
| 262 | $SCH_3$ | $CH_3$ | $C_6H_5$-O- | CH | $(CH_2)_4$ | |
| 263 | $SCH_3$ | $CH_3$ | 2-Cl-$C_6H_5$-O- | CH | $(CH_2)_5$ | |
| 264 | $SCH_3$ | $CH_3$ | 2-Cl-$C_6H_5$-O- | CH | $(CH_2)_6$ | |
| 265 | $NHCH_3$ | $CH_3$ | $C_6H_5$-O- | CH | $(CH_2)_4$ | |
| 266 | $NHCH_3$ | $CH_3$ | 2-Cl-$C_6H_4$-O- | CH | $(CH_2)_5$ | |
| 267 | $NHCH_3$ | $CH_3$ | 2-Cl-$C_6H_4$-O- | CH | $(CH_2)_6$ | |
| 268 | $N(CH_3)_2$ | $CH_3$ | $C_6H_5$-O- | CH | $(CH_2)_4$ | |
| 269 | $N(CH_3)_2$ | $CH_3$ | 2-Cl-$C_6H_4$-O- | CH | $(CH_2)_5$ | |
| 270 | $N(CH_3)_2$ | $CH_3$ | 2-Cl-$C_6H_4$-O- | CH | $(CH_2)_6$ | |
| 271 | $OCH_3$ | $CH_3$ | $C_6H_5$-O- | N | $(CH_2)_4$ | |
| 272 | $OCH_3$ | $CH_3$ | 2-Cl-$C_6H_4$-O- | N | $(CH_2)_5$ | |
| 273 | $OCH_3$ | $CH_3$ | 2-Cl-$C_6H_4$-O- | N | $(CH_2)_6$ | |
| 274 | $NHCH_3$ | $CH_3$ | $C_6H_5$-O- | N | $(CH_2)_4$ | |
| 275 | $NHCH_3$ | $CH_3$ | 2-Cl-$C_6H_4$-O- | N | $(CH_2)_5$ | |
| 276 | $NHCH_3$ | $CH_3$ | 2-Cl-$C_6H_4$-O- | N | $(CH_2)_6$ | |
| 277 | $N(CH_3)_2$ | $CH_3$ | $C_6H_5$-O- | N | $(CH_2)_4$ | |
| 278 | $N(CH_3)_2$ | $CH_3$ | 2-Cl-$C_6H_4$-O- | N | $(CH_2)_5$ | |
| 279 | $N(CH_3)_2$ | $CH_3$ | 2-Cl-$C_6H_4$-O- | N | $(CH_2)_6$ | |
| 283 | $OCH_3$ | $CH_3$ | $C_6H_5$-O- | N | $(CH_2)_2$ | 98-101°C (E/Z=2/1) |
| 284 | $OCH_3$ | $CH_3$ | 2-Cl-$C_6H_4$-O- | N | $(CH_2)_2$ | |
| 285 | $OCH_3$ | $CH_3$ | 3-Cl-$C_6H_4$-O- | N | $(CH_2)_2$ | |
| 286 | $OCH_3$ | $CH_3$ | 2,6-$Cl_2$-$C_6H_3$-O- | N | $(CH_2)_4$ | |
| 287 | $OCH_3$ | $CH_3$ | 4-$OCH_3$-$C_6H_4$-O- | N | $(CH_2)_4$ | |
| 288 | $OCH_3$ | $CH_3$ | 2-F-$C_6H_4$-O- | N | $(CH_2)_5$ | |
| 289 | $OCH_3$ | $CH_3$ | 3,5-$CL_2$-$C_6H_3$-O- | N | $(CH_2)_{10}$ | |

Tabelle 3:

NMR-Daten ausgewählter Verbindungen aus den Tabellen 1 und 2. Die chemische Verschiebung (δ) wird in ppm relativ zu Tetramethylsilan angegeben. Als Lösungsmittel dient $CDCl_3$.

Verbindung Nr. 66

2.17 (q,2H); 3.60 (s,3H); 3.71 (s,3H); 4.11 (m,4H); 7.08 (m,9H); 7.47 (s,1H).

Verbindung Nr. 97

2.88 (m,4H); 3.68 (s,3H); 3.77 (s,3H); 4.00 (m,4H); 7.08 (m,9H); 7.48 (s,1H).

Verbindung Nr. 140

1.63 (m,2H); 2.85 (m,4H); 3.66 (s,3H); 3.80 (s,3H); 3.98 (t,2H); 4.05 (t,2H); 7.12 (m,8H); 7.50 (s,1H).

Verbindung Nr. 163

1.50 (m,4H); 1.75 (m,4H); 3.67 (s,3H); 3.78 (s,3H); 3.94 (t,2H); 4.03 (t,2H); 7.12 (m,8H); 7.49 (s,1H).

Verbindung Nr. 195

1.41 (m, 6H); 1.75 (m, 4H); 2.28 (s, 3H); 3.68 (s, 3H); 3.80 (s, 3H); 3.90 (t, 2H); 3.93 (t, 2H); 7.00 (m, 8H); 7.48 (s, 1H).

Verbindung Nr. 209

1.36 (m, 8H); 1.72 (m, 4H); 3.65 (s, 3H); 3.75 (s, 3H); 3.89 (t, 2H); 3.92 (t, 2H); 6.79 - 7.28 (m, 9H); 7.45 (s, 1H).

Verbindung Nr. 232

1.33 (m, 10H); 1.73 (m, 4H); 3.68 (s, 3H); 3.80 (s, 3H); 3.90 (t, 2H); 3.94 (t, 2H); 6.84 - 7.70 (m, 9H); 7.48 (s, 1H).

Verbindung Nr. 233

1.32 (m, 12H); 1.75 (m, 4H); 3.68 (s, 3H); 3.80 (s, 3H); 3.92 (t, 2H); 3.95 (t, 2H); 6.87 - 7.31 (m, 9H); 7.50 (s, 1H).

Verbindung Nr. 282

1.31 (m, 16H); 1.73 (m, 4H); 3.69 (s, 3H); 3.80 (s, 3H); 3.91 (t, 2H); 3.93 (t, 2H); 6.77 - 7.28 (m, 8H); 7.48 (s, 1H).

Verbindung 283

3.75, 3.80 (2s, 3H); 4.00, 4.05 (2s, 3H); 4.30 (m, 4H); 6.90 - 7.50 (m, 9H).

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,

Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 66 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 97 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 140 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 164 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 163 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 66 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 97 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 140 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 163 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:
Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N'-propylen-bis-dithiocarbamat),
Zink-(N,N'-propylen-bis-dithiocarbamat),
N,N'-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester
heterocyclische Substanzen, wie
2-Heptadecyl-2-imidazolin-acetat,
2,4-Dichlor-6-(o-chloranilino)-s-triazin,
O,O-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N',N'-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,

N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan ,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N'-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid, Hexachlorbenzol,
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3--chlor-2-aminopyridin,
1-((bis-(4-Fluorphenyl)-methylsilyl)-methyl)-1H-1,2,4-triazol.

Anwendungsbeispiele

Als Vergleichswirkstoff wurde N-Tridecyl-2,6-dimethylmorpholin (A) - bekannt aus DE 1 164 152 - benutzt.

Anwendungsbeispiel 1

Wirksamkeit gegen Pyrenophora teres

Gerstenkeimlinge der Sorte "Igri" wurden im Zweiblattstadium mit wäßrigen Suspensionen, die 80 % Gew.% Wirkstoff und 20 % Emulgator in der Trockensubstanz enthielten, tropfnaß gespritzt. Nach 24 Stunden wurden die Pflanzen mit einer Sporensuspension des Pilzes Pyrenopthora teres inokuliert und für 48 Stunden in eine Klimakammer mit hoher Luftfeuchtigkeit bei 18°C gestellt. Anschließend wurden die Pflanzen im Gewächshaus bei 20 - 22°C und 70 % relativer Luftfeuchtigkeit für weitere 5 Tage kultiviert. Dann wurde das Ausmaß der Symptomentwicklung ermittelt.

Das Ergebnis zeigt, daß die Wirkstoffe 97, 140 und 164 bei der Anwendung als 0,05 %ige Spritzbrühe eine bessere fungizide Wirkung (97 %) zeigen als der bekannte Vergleichswirkstoff A (70 %).

Anwendungsbeispiel 2

Wirksamkeit gegen Phytophthora infestans in Tomaten

Blätter von Topfpflanzen der Sorte "Große Fleischtomate" wurden mit wäßriger Spritzbrühe, die 80 % Wirkstoff und 20 % Emulgiermittel in der Trockensubstanz enthielt, besprüht. Nach 24 Stunden wurden die Blätter mit einer Zoosporenaufschwemmung des Pilzes Phytophthora infestans infiziert. Die Pflanzen wurden dann in eine wasserdampfgesättigten Kammer bei Temperaturen zwischen 16 und 18°C gestellt. Nach 6 Tagen hatte sich die Krankheit auf den unbehandelten, jedoch infizierten Kontrollpflanzen so stark entwickelt, daß die fungizide Wirksamkeit der Substanzen beurteilt werden konnte.

Das Ergebnis zeigt, daß die Wirkstoffe 66, 140, 163 und 164 bei der Anwendung als 0,025 %ige Spritzbrühe eine bessere fungizide Wirkung (95 %) zeigen als der bekannte Vergleichswirkstoff A (55 %).

Anwendungsbeispiel 3

Wirksamkeit gegen Plasmopara viticola

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus mit Temperaturen zwischen 20 und 30°C aufgestellt. Nach dieser Zeit wurden die Pflanzen zur Beschleunigung des Sporangienträgerausbruches abermals für 16 Stunden in der feuchten Kammer aufgestellt. Dann erfolgte die Beurteilung des Ausmaßes des Pilzausbruches auf den Blattunterseiten.

Das Ergebnis zeigt, daß die Wirkstoffe 66, 97, 140, 163 und 164 bei der Anwendung als 0,05%ige Spritzbrühe eine bessere fungizide Wirkung (97%) zeigen als der bekannte Vergleichswirkstoff A (35%).

## Patentansprüche

1. Ortho-substituierte Phenolether der allgemeinen Formel I

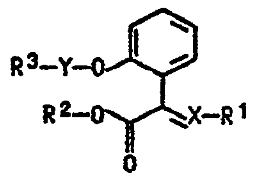

I,

in der

$R^1$ $C_1$-$C_4$-Alkyl, $C_1$-$C_4$-Alkoxy, $C_1$-$C_4$-Alkylthio oder gegebenenfalls durch $C_1$-$C_4$-Alkyl einfach oder doppelt substituiertes Amino bedeutet,

$R^2$ $C_1$-$C_4$-Alkyl bedeutet,

$R^3$ Aryloxy, Arylthio oder Arylalkoxy bedeutet, wobei der aromatische Ring gegebenenfalls durch einen oder mehrere der folgenden Reste substituiert ist: Halogen, $C_1$-$C_6$-Alkyl, $C_1$-$C_6$-Alkoxy, $C_1$-$C_4$-Alkylthio, $C_1$-$C_2$-Halogenalkyl, Aryl, Aryl-$C_1$-$C_2$-alkoxy, $C_1$-$C_4$-Alkylcarbonyl, durch $C_1$-$C_4$-Alkyl einfach oder doppelt substituiertes Amino, Cyano, Nitro,

X CH oder N bedeutet,

Y einen gegebenenfalls ungesättigten $C_2$-$C_{12}$-Alkylenrest bedeutet.

2. Verfahren zur Bekämpfung von Pilzen, dadurch gekennzeichnet, daß man die Pilze oder die von Pilzbefall bedrohten Materialien, Pflanzen, Saatgüter oder den Erdboden mit einer fungizid wirksamen Menge eines ortho-substituierten Phenolethers der allgemeinen Formel I

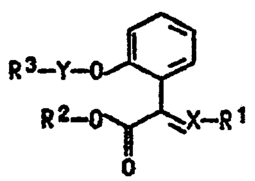

I,

behandelt, in der

$R^1$ $C_1$-$C_4$-Alkyl, $C_1$-$C_4$-Alkoxy, $C_1$-$C_4$-Alkylthio oder gegebenenfalls durch $C_1$-$C_4$-Alkyl einfach oder doppelt substituiertes Amino bedeutet,

$R^2$ $C_1$-$C_4$-Alkyl bedeutet,

$R^3$ Aryloxy, Arylthio oder Arylalkoxy bedeutet, wobei der aromatische Ring gegebenenfalls durch einen oder mehrere der folgenden Reste substituiert ist: Halogen, $C_1$-$C_6$-Alkyl, $C_1$-$C_6$-Alkoxy, $C_1$-$C_4$-Alkylthio, $C_1$-$C_2$-Halogenalkyl, Aryl, Aryl-$C_1$-$C_2$-alkoxy, $C_1$-$C_4$-Alkylcarbonyl, durch $C_1$-$C_4$-Alkyl einfach oder doppelt substituiertes Amino, Cyano, Nitro,

X CH oder N bedeutet,

Y einen gegebenenfalls ungesättigten $C_2$-$C_{12}$-Alkylenrest bedeutet.

3. Fungizid, enthaltend einen inerten Trägerstoff und einen ortho-substituierten Phenolether der allgemeinen Formel I

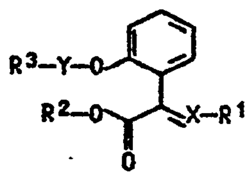

I,

in der

$R^1$ $C_1$-$C_4$-Alkyl, $C_1$-$C_4$-Alkoxy, $C_1$-$C_4$-Alkylthio oder gegebenenfalls durch $C_1$-$C_4$-Alkyl einfach oder doppelt substituiertes Amino bedeutet,

$R^2$ $C_1$-$C_4$-Alkyl bedeutet,

$R^3$ Aryloxy, Arylthio oder Arylalkoxy bedeutet, wobei der aromatische Ring gegebenenfalls durch einen oder mehrere der folgenden Reste substituiert ist: Halogen, $C_1$-$C_6$-Alkyl, $C_1$-$C_6$-Alkoxy, $C_1$-$C_4$-Alkylthio, $C_1$-$C_2$-Halogenalkyl, Aryl, Aryl-$C_1$-$C_2$-alkoxy, $C_1$-$C_4$-Alkylcarbonyl, durch $C_1$-$C_4$-Alkyl einfach oder doppelt substituiertes Amino, Cyano, Nitro,

X CH oder N bedeutet,

Y einen gegebenenfalls ungesättigten $C_2$-$C_{12}$-Alkylenrest bedeutet.

4. Verbindung gemäß Anspruch 1, dadurch gekennzeichnet, daß $R^1$ Methoxy, $R^2$ Methyl, $R^3$ Phenoxy, Y Butylen und X den Rest CH bedeuten.

5. Verbindung gemäß Anspruch 1, dadurch gekennzeichnet, daß $R^1$ Methoxy, $R^2$ Methyl, $R^3$ 2-Chlorphenoxy, Y Pentylen und X den Rest CH bedeuten.

6. Verbindung gemäß Anspruch 1, dadurch gekennzeichnet, daß $R^1$ Methoxy, $R^2$ Methyl, $R^3$ 2-Chlorphenoxy, Y Hexylen und X den Rest CH bedeuten.

7. Verbindung gemäß Anspruch 1, dadurch gekennzeichnet, daß $R^1$ Methoxy, $R^2$ Methyl, $R^3$ Phenoxy, Y Propylen und X den Rest CH bedeuten.

8. Ortho-substituierte Phenolether der allgemeinen Formel II,

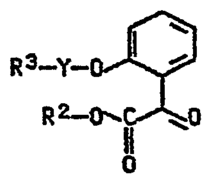

II,

in der

$R^2$ $C_1$-$C_4$-Alkyl bedeutet,

$R^3$ Aryloxy, Arylthio oder Arylalkoxy bedeutet, wobei der aromatische Ring gegebenenfalls durch einen oder mehrere der folgenden Reste substituiert ist: Halogen, $C_1$-$C_6$-Alkyl, $C_1$-$C_6$-Alkoxy, $C_1$-$C_4$-Alkylthio, $C_1$-$C_2$-Halogenalkyl, Aryl, Aryl-$C_1$-$C_2$-alkoxy, $C_1$-$C_4$-Alkylcarbonyl, durch $C_1$-$C_4$-Alkyl einfach oder doppelt substituiertes Amino, Cyano, Nitro,

Y einen gegebenenfalls ungesättigten $C_2$-$C_{12}$-Alkylenrest bedeutet.

## Claims

1. An ortho-substituted phenol ether of the general formula I

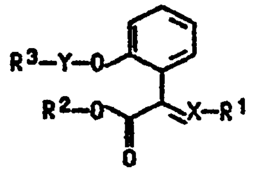

I,

where

$R^1$ is $C_1$–$C_4$-alkyl, $C_1$–$C_4$-alkoxy, $C_1$–$C_4$-alkylthio, or amino which is unsubstituted or mono- or disubstituted by $C_1$–$C_4$-alkyl,

$R^2$ is $C_1$–$C_4$-alkyl,

$R^3$ is aryloxy, arylthio or arylalkoxy, the aromatic ring being unsubstituted or substituted by one or more of the following radicals: halogen, $C_1$–$C_6$-alkyl, $C_1$–$C_6$-alkoxy, $C_1$–$C_4$-alkylthio, $C_1$–$C_2$-haloalkyl, aryl, aryl-$C_1$–$C_2$-alkoxy, $C_1$–$C_4$-alkylcarbonyl, mono- or di-$C_1$–$C_4$-alkyl-substituted amino, cyano, nitro,

X is CH or N, and

Y is saturated or unsaturated $C_2$–$C_{12}$-alkylene.

2. A process for combating fungi, wherein the fungi, or the materials, plants or seed threatened by fungus attack or the soil are treated with a fungicidally effective amount of an ortho-substituted phenol ether of the general formula I

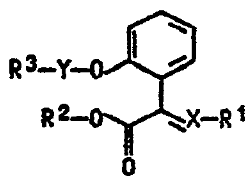

I,

where

$R^1$ is $C_1$–$C_4$-alkyl, $C_1$–$C_4$-alkoxy, $C_1$–$C_4$-alkylthio, or amino which is unsubstituted or mono- or disubstituted by $C_1$–$C_4$-alkyl,

$R^2$ is $C_1$–$C_4$-alkyl,

$R^3$ is aryloxy, arylthio or arylalkoxy, the aromatic ring being unsubstituted or substituted by one or more of the following radicals: halogen, $C_1$–$C_6$-alkyl, $C_1$–$C_6$-alkoxy, $C_1$–$C_4$-alkylthio, $C_1$–$C_2$-haloalkyl, aryl, aryl-$C_1$–$C_2$-alkoxy, $C_1$–$C_4$-alkylcarbonyl, mono- or di-$C_1$–$C_4$-alkyl-substituted amino, cyano, nitro,

X is CH or N, and

Y is saturated or unsaturated $C_2$–$C_{12}$-alkylene.

3. A fungicide containing an inert carrier and an ortho-substituted phenol ether of the general formula I

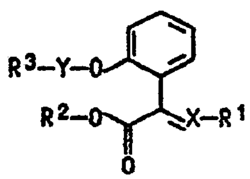

I,

where

$R^1$ is $C_1$–$C_4$-alkyl, $C_1$–$C_4$-alkoxy, $C_1$–$C_4$-alkylthio, or amino which is unsubstituted or mono- or disubstituted by $C_1$–$C_4$-alkyl,

$R^2$ is $C_1$–$C_4$-alkyl,

$R^3$ is aryloxy, arylthio or arylalkoxy, the aromatic ring being unsubstituted or substituted by one or more of the following radicals: halogen, $C_1$–$C_6$-alkyl, $C_1$–$C_6$-alkoxy, $C_1$–$C_4$-alkylthio, $C_1$–$C_2$-haloalkyl, aryl, aryl-$C_1$–$C_2$-alkoxy, $C_1$–$C_4$-alkylcarbonyl, mono- or di-$C_1$–$C_4$-alkyl-substituted amino, cyano, nitro,

X is CH or N, and

Y is saturated or unsaturated $C_2$–$C_{12}$-alkylene.

4. A compound as claimed in claim 1, where $R^1$ is methoxy, $R^2$ is methyl, $R^3$ is phenoxy, Y is butylene and X is CH.

5. A compound as claimed in claim 1, where $R^1$ is methoxy, $R^2$ is methyl, $R^3$ is 2-chlorophenoxy, Y is pentylene and X is CH.

6. A compound as claimed in claim 1, where $R^1$ is methoxy, $R^2$ is methyl, $R^3$ is 2-chlorophenoxy, Y is hexylene and X is CH.

7. A compound as claimed in claim 1, where $R^1$ is methoxy, $R^2$ is methyl, $R^3$ is phenoxy, Y is propylene and X is CH.

8. An ortho-substituted phenol ether of the general formula II,

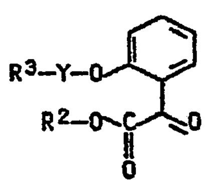

II,

where

$R^2$ is $C_1$–$C_4$-alkyl,

$R^3$ is aryloxy, arylthio or arylalkoxy, the aromatic ring being unsubstituted or substituted by one or more of the following radicals: halogen, $C_1$–$C_6$-alkyl, $C_1$–$C_6$-alkoxy, $C_1$–$C_4$-alkylthio, $C_1$–$C_2$-haloalkyl, aryl, aryl-$C_1$–$C_2$-alkoxy, $C_1$–$C_4$-alkylcarbonyl, mono- or di-$C_1$–$C_4$-alkyl-substituted amino, cyano, nitro,

Y is saturated or unsaturated $C_2$–$C_{12}$-alkylene.

## Revendications

1. Ether phénolique ortho-substitué de la formule générale

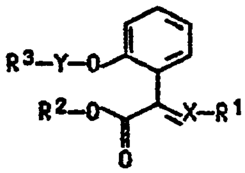

I,

dans laquelle

$R^1$ représente alkyle en $C_1$–$C_4$, alcoxy en $C_1$–$C_4$, alkylthio en $C_1$–$C_4$, ou amino éventuellement substitué une ou deux fois par alkyle en $C_1$–$C_4$

$R^2$ représente alkyle en $C_1$–$C_4$

$R^3$ représente aryloxy, arylthio ou arylalcoxy, le noyau aromatique étant éventuellement substitué par un ou plusieurs des restes suivants: halogène, alkyle en $C_1$–$C_6$, alcoxy en $C_1$–$C_6$, alkylthio en $C_1$–$C_4$, halogénalkyle en $C_1$–$C_2$, aryle, aryl-alcoxy en $C_1$–$C_2$, alkyle (en $C_1$–$C_4$) carbonyle, amino substitué une ou deux fois par alkyle en $C_1$–$C_4$, cyano, nitro,

X représente CH ou N

Y représente un reste alkylène en $C_2$–$C_{12}$ éventuellement substitué.

2. Procédé de lutte contre les champignons, caractérisé par le fait qu'on traite les champignons ou les matériaux, plantes, semences ou les sols avec une quantité efficace au point de vue herbicide d'un éther phénolique ortho-substitué de la formule générale I

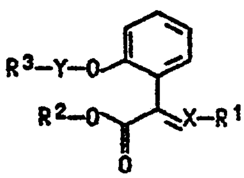

I,

dans laquelle

$R^1$ représente alkyle en $C_1$–$C_4$, alcoxy en $C_1$–$C_4$, alkylthio en $C_1$–$C_4$, ou amino éventuellement substitué une ou deux fois par alkyle en $C_1$–$C_4$

$R^2$ représente alkyle en $C_1$–$C_4$

$R^3$ représente aryloxy, arylthio ou arylalcoxy, le noyau aromatique étant éventuellement substitué par un ou plusieurs des restes suivants: halogène, alkyle en $C_1$–$C_6$, alcoxy en $C_1$–$C_6$, alkylthio en $C_1$–$C_4$, halogénalkyle en $C_1$–$C_2$, aryle, aryl-alcoxy en $C_1$–$C_2$, alkyle (en $C_1$–$C_4$) carbonyle, amino substitué une ou deux fois par alkyle en $C_1$–$C_4$, cyano, nitro,

X représente CH ou N

Y représente un reste alkylène en $C_2$–$C_{12}$ éventuellement substitué.

3. Fongicide contenant un support inerte et un éther phénolique ortho-substitué de la formule générale I

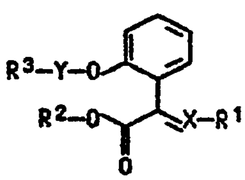

I,

dans laquelle

$R^1$ représente alkyle en $C_1$–$C_4$, alcoxy en $C_1$–$C_4$, alkylthio en $C_1$–$C_4$, ou amino éventuellement substitué une ou deux fois par alkyle en $C_1$–$C_4$

$R^2$ représente alkyle en $C_1$–$C_4$

$R^3$ représente aryloxy, arylthio ou arylalcoxy, le noyau aromatique étant éventuellement substitué par un ou plusieurs des restes suivants: halogène, alkyle en $C_1$–$C_6$, alcoxy en $C_1$–$C_6$, alkylthio en $C_1$–$C_4$, halogénalkyle en $C_1$–$C_2$, aryle, aryl-alcoxy en $C_1$–$C_2$, alkyle (en $C_1$–$C_4$) carbonyle, amino substitué une ou deux fois par alkyle en $C_1$–$C_4$, cyano, nitro,

X représente CH ou N

Y représente un reste alkylène en $C_2$–$C_{12}$ éventuellement substitué.

4. Composé selon la revendication 1, caractérisé par le fait que $R^1$ représente méthoxy, $R^2$, méthyle, $R^3$, phénoxy, Y, butylène et X le reste CH.

5. Composé selon la revendication 1, caractérisé par le fait que $R^1$ représente methoxy, $R^2$, méthyle, $R^3$, 2-chlorophénoxy, Y pentylène et X le reste CH.

6. Composé selon la revendication 1, caractérisé par le fait que $R^1$ représente méthoxy, $R^2$, methyle, $R^3$, 2-chlorophénoxy, Y, héxylène et X le reste CH.

7. Composé selon la revendication 1, caractérisé par le fait que $R^1$ représente méthoxy, $R^2$, méthyle, $R^3$, phénoxy, Y, propylène et X le reste CH.

8. Ether phénolique ortho-substitué de la formule générale II

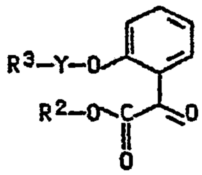

II.

dans laquelle

$R^2$ représente alkyle en $C_1$–$C_4$

$R^3$ représente aryloxy, arylthio ou arylalcoxy, le noyau aromatique étant éventuellement substitué par un ou plusieurs des restes suivants: halogène, alkyle en $C_1$–$C_6$, alcoxy en $C_1$–$C_6$, alkylthio en $C_1$–$C_4$, halogénalkyle en $C_1$–$C_2$, aryle, aryl-alcoxy en $C_1$–$C_2$, alkyle (en $C_1$–$C_4$) carbonyle, amino substitué une ou deux fois par alkyle en $C_1$–$C_4$, cyano, nitro,

Y représente un reste alkylique en $C_2$–$C_{12}$ éventuellement substitué.